# EUROPEAN PATENT APPLICATION

(11) **EP 1 862 191 A1**
(43) Date of publication of application: **05.12.2007**
(21) Application number: 06126706.8
(22) Date of filing: 20.12.2006
(51) Int. Cl.: A61M 5/32

(54) **Safety syringe device**

(71) Applicant: HUANG, Hung-Chi, Taichung City (TW)
(72) Inventor: HUANG, Hung-Chi, Taichung City (TW)
(74) Representative: Viering, Jentschura & Partner

(57) **Abstract**

A safety syringe device (1) includes a barrel (10), a needle unit connected to an end of the barrel, a plunger (20) movably inserted into a passage (12) of the barrel, a stopper (21) mounted to a tubular portion (30) extending from the plunger and out from the stopper. The needle unit has a connection member (41) received in the barrel and a needle cannula (42) is connected to a first end of the connection member. A tube (441) is located at a center of the connection member and an annular recess (443) is defined between the tube and a wall of the second end of the connection member. The tube is engaged with a recess (31) of the tubular portion on the plunger and a flange (32) of the tubular portion is engaged with the annular recess so as to connect the plunger and the connection member. Therefore, the connection member and the needle cannula are pulled into the barrel when the plunger is pulled backward.

## Description

### BACKGROUND OF THE INVENTION

### (1) FIELD OF THE INVENTION

The present invention relates to a safety syringe device wherein the needle hub is pulled back into the barrel after use.

### (2) DESCRIPTION OF THE PRIOR ART

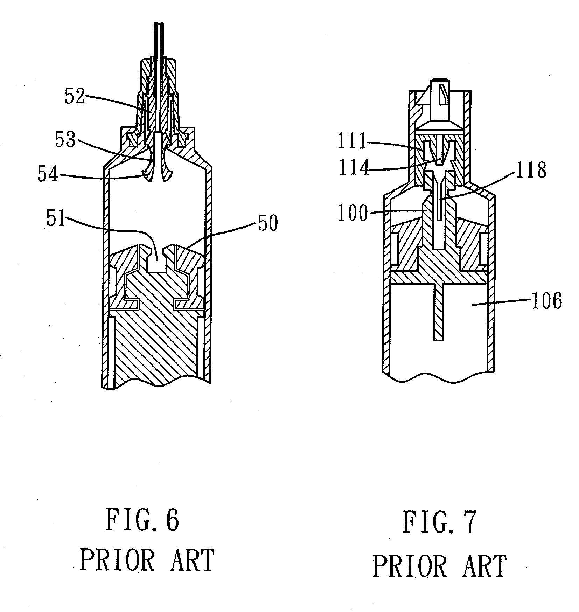

A conventional safety syringe device is shown in Fig. 6 and generally includes barrel with a plunger inserted therein and a stopper 50 is connected to an end of the plunger so as to force medicine in the barrel out from the needle cannula connected to a connection member 52 engaged with a hole in the front end of the barrel. The stopper 50 includes a recess 51 defined in a front end thereof and the connection member 52 has two legs 53 extending therefrom which extend into the barrel. Each leg 53 has an enlarged portion 54 which is supposed to expand the recess 51 to allow the two legs 53 secured to the recess 51. However, it is experienced that the legs 53 cannot successfully engaged with the recess 51 so that the connection member 52 together with the needle cannula are not pulled back into the barrel when pulling the plunger.

As shown in Fig. 7 which shows another conventional safety syringe device wherein the stopper 100 on the plunger 106 has an extension portion 118 which has two side blocks so as to be expand the notch of the connection member 111. A cone-shaped member extends from an inner end of the notch of the connection member 111 and the two side blades can be opened wide by the cone-shaped member and then are engaged with two hooks extending from the inner periphery of the notch to connect the stopper 100 and the connection member111.

It is noted that both of the conventional syringe devices require a certain force to engage the two legs 53 with the recess 51, and to engage the side blocks with the notch and the hooks. The user might push the plunger too hard in order to connect the connection member with the stopper, and this might lead the connection member together with the needle cannula to be pushed out from the barrel.

The present invention intends to provide a safety syringe device wherein the stopper on the plunger is easily connected to the connection member in the barrel so as to avoid the inherent shortcomings of the conventional safety syringe devices.

### SUMMARY OF THE INVENTION

The present invention relates to a safety syringe device which comprises a barrel having a needle unit connected to an end thereof and a passage is defined axially through the barrel. A plunger is movably inserted into the passage of the barrel and a tubular portion is connected to a first end of the plunger. A recess is defined in an end of the tubular portion and a flange extends outward from a periphery of the tubular portion. A stopper is mounted to the tubular portion and a distal end of the tubular portion extends out from the stopper. The needle unit has a connection member which has a needle cannula connected to a first end thereof and a cooperation portion is formed on a second end of the connection member. A tube is located at a center of the connection member and a path is defined axially through the tube. An annular recess is defined between the tube and a wall of the second end of the connection member. The tube is engaged with the recess of the tubular portion on the plunger and the flange is engaged with the annular recess when the tubular portion is engaged with the cooperation portion of the connection member so as to connect the plunger and the connection member, so that the connection member and the needle cannula are pulled into the barrel when the plunger is pulled backward.

The present invention will become more obvious from the following description when taken in connection with the accompanying drawings which show, for purposes of illustration only, a preferred embodiment in accordance with the present invention.

### BRIEF DESCRIPTION OF THE DRAWINGS

Fig. 1 is an exploded view to show the safety syringe device of the present invention;
Fig. 2 is a cross sectional view to show the plunger is connected to the connection member;
Fig. 3 is a cross sectional view of the connection of the plunger and the connection member;
Fig. 4 shows that the plunger is broken after the connection member and the needle cannula with the protection cap are pulled into the barrel;
Fig. 5 shows that the needle cannula without the protection cap is pulled into the barrel;
Fig. 6 is a cross sectional view to show the first conventional safety syringe device, and
Fig. 7 is a cross sectional view to show the second conventional safety syringe device.

### DETAILED DESCRIPTION OF THE PREFERRED EMBODIMENTS

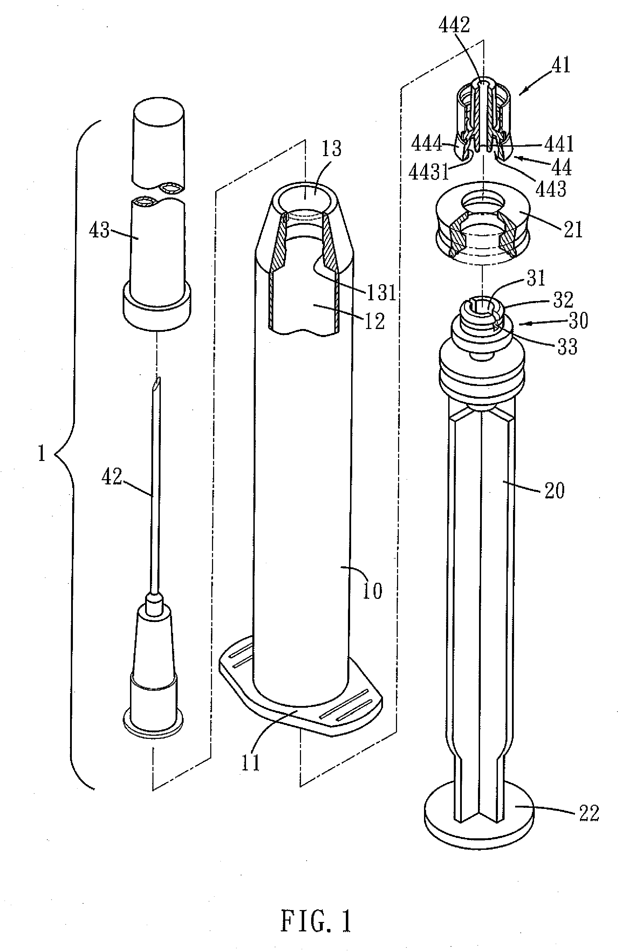

Referring to Fig. 1, the safety syringe device 1 of the present invention comprises a barrel 10 having a needle unit connected to a first end thereof and a finger flange 11 is connected to a second end of the barrel 10, A passage 12 is defined axially through the barrel 10 and an opening 13 is defined through the first end of the barrel 10 and communicates with the passage 12. The size of the opening 13 is smaller than the size of the passage 12. The passage 12 includes an engaging space which is located close to the first end of the barrel 10 and includes a tapered inner periphery 131.

A plunger 20 is movably inserted into the passage 12 of the barrel 10 and a tubular portion 30 is axially connected to a first end of the plunger 20. A recess 31 is defined in an end of the tubular portion 30 and a flange 32 extends outward from a periphery of the tubular portion 30. A slot 33 is defined radially through a wall of the tubular portion 30 and define the tubular portion 30 into two parts. A thumb rest 22 connected to a second end of the plunger 20.

A stopper 21 is mounted to the tubular portion 30 and a distal end of the tubular portion 30 including the two parts defined by the slot 33 extends out from the stopper 21. An outer periphery of the stopper 21 is in contact with an inner periphery of the passage 12.

The needle unit has a connection member 41 which has a needle cannula 42 connected to a first end thereof and a cooperation portion 44 is formed on a second end of the connection member 41. The cooperation portion 44 of the connection member 41 has a tapered outer periphery 444 which is engaged with the tapered inner periphery 131 of the engaging space. A tube 441 is located at a center of the connection member 41 and a path 442 is defined axially through the tube 441. An annular recess 443 is defined between the tube 441 and a wall of the second end of the connection member 41. A groove 4431 is defined in an inner periphery of the annular recess 443 of the connection member 41. The tube 441 includes a tapered section which is located in the annular recess 443 and a size of a distal end of the tapered section of the tube 441 is smaller than an inner diameter of the recess 31 of the tubular portion 30.

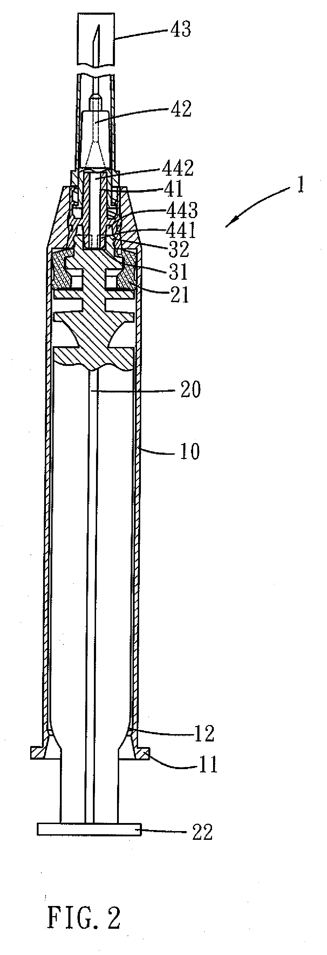

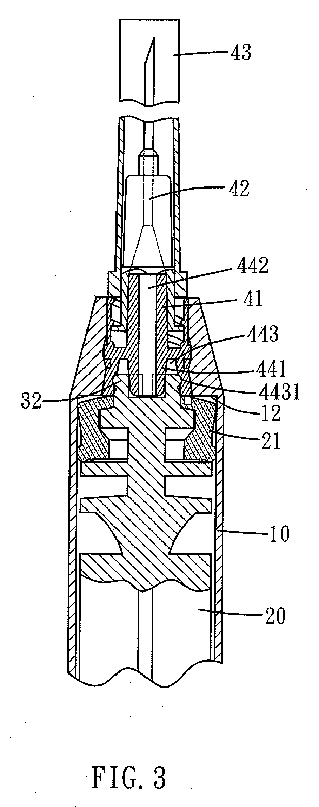

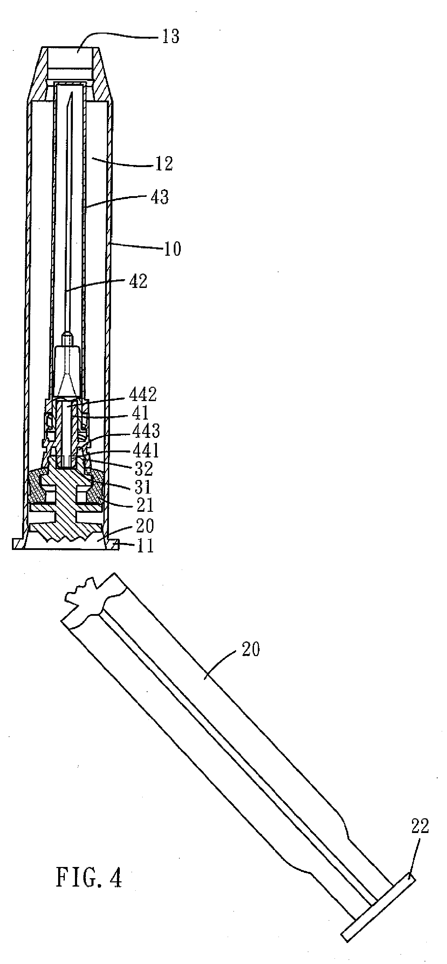

Referring to Figs. 2 and 3, the user pushes the plunger 20 toward the needle unit to eject medicine out from the needle cannula 42 and after the rejection, the tube 441 is engaged with the recess 31 of the tubular portion 30 on the plunger 20 and the two parts defined by the slot 33 of the tubular portion 30. The two parts are pushed outward by the tapered section of the tube 441 when the tube 441 is inserted into the recess 31. The flange 32 is then engaged with the groove 4431 in the annular recess 443 of the cooperation portion 44 of the connection member 41 so as to connect the plunger 20 and the connection member 41. When the plunger 20 is pulled backward, the connection member 41 and the needle cannula 42 are pulled into the barrel 10. It is noted that the size of the opening 13 can be set such that the protection cap 43 mounted on the needle hub of the needle unit can also be pulled into the barrel 10 as shown in Fig. 4. The plunger 20 can further be broken and this is well known in the art.

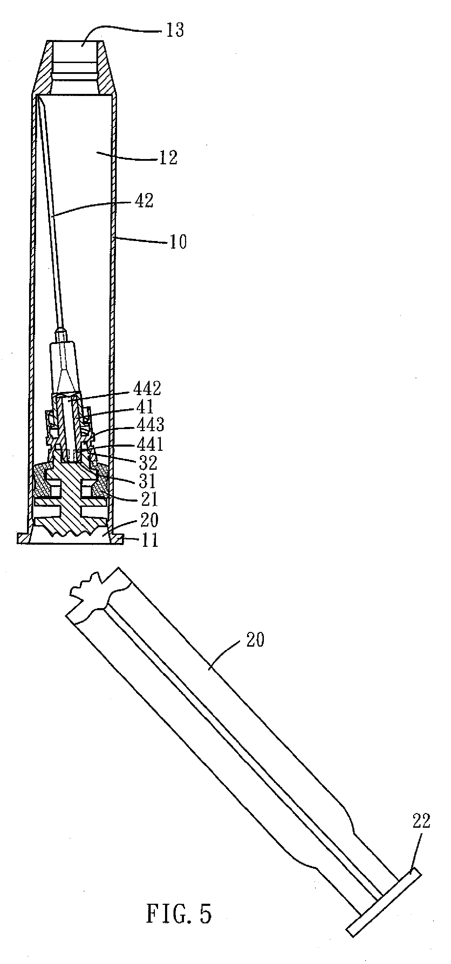

As shown in Fig. 5, if the protection cap is removed from the needle cannula 42 and the needle cannula 42 is pulled into the barrel 10, the underside of the cooperation portion 44 of the connection member 41 is in contact with the tapered top surface of the stopper 21 so that the needle cannula 42 tilts to further avoid the needle cannula 42 from extending from the opening 13 of the barrel 10.

The connection member 41, the tube 441, the groove 4431, and the tubular portion 30 can be easily made so that the manufacturing cost can be kept at low standard. The tapered section of the tube 441 is easily inserted into the recess 31 of the tubular portion 30 and easily pushes the two parts of the tubular portion 30 outward to engage with the groove 4431. Therefore, the users do not need to push the plunger 20 hard to connect the plunger 20 to the connection member 41.

While we have shown and described the embodiment in accordance with the present invention, it should be clear to those skilled in the art that further embodiments may be made without departing from the scope of the present invention.

## Claims

1. A safety syringe device comprising:
a barrel having a needle unit connected to a first end thereof and a finger flange connected to a second end of the barrel, a passage defined axially through the barrel and an opening defined through the first end of the barrel and communicating with the passage, a size of the opening being smaller than a size of the passage;
a plunger movably inserted into the passage of the barrel and a tubular portion connected to a first end of the plunger, a recess defined in an end of the tubular portion and a flange extending outward from a periphery of the tubular portion, a stopper mounted to the tubular portion and a distal end of the tubular portion extending out from the stopper, an outer periphery of the stopper being in contact with an inner periphery of the passage, a thumb rest connected to a second end of the plunger, and
the needle unit having a connection member which has a needle cannula connected to a first end thereof and a cooperation portion is formed on a second end of the connection member, a tube located at a center of the connection member and a path defined axially through the tube, an annular recess defined between the tube and a wall of the second end of the connection member, the tube being engaged with the recess of the tubular portion on the plunger and the flange being engaged with the annular recess when the tubular portion engaged with the cooperation portion of the connection member so as to connect the plunger and the connection member, the connection member and the needle cannula being pulled into the barrel when the plunger is pulled backward.

2. The device as claimed in claim 1, wherein the tube includes a tapered section which is located in the annular recess and a size of a distal end of the tapered section of the tube is smaller than an inner diameter of the recess of the tubular portion, a slot is defined radially through a wall of the tubular portion and define the tubular portion into two parts which are pushed outward by the tapered section of the tube when the tube is inserted into the recess.

3. The device as claimed in claim 1, wherein a groove is defined in an inner periphery of the annular recess of the connection member and the flange of the tubular portion is engaged with the groove when the tubular portion is connected with the connection member.

4. The device as claimed in claim 1, wherein the passage includes an engaging space which is located close to the first end of the barrel and includes a tapered inner periphery, the cooperation portion of the connection member has a tapered outer periphery which is engaged with the tapered inner periphery of the engaging space.
